# EUROPEAN PATENT SPECIFICATION

(11) **EP 2 567 657 B1**
(45) Date of publication and mention of the grant of the patent: **27.11.2019**
(21) Application number: 12183353.7
(22) Date of filing: 06.09.2012
(51) Int. Cl.: A61B 5/04, A61B 5/0428, A61B 5/053

(54) **Synchronization and communication bus for biopotential and bioimpedance measurement systems.**
Synchronisations- und Kommunikationsbus für Biopotenzial- und Bioimpedanzmesssysteme
Bus de communication et de synchronisation pour systèmes de mesure de bioimpédance et de biopotentiel

(30) Priority: 07.09.2011 CH 14712011
(43) Date of publication of application: 13.03.2013
(73) Proprietor: CSEM Centre Suisse d'Electronique et de Microtechnique SA - Recherche et Développement, 2002 Neuchâtel (CH)
(72) Inventor: Chételat, Olivier, 1588 Cudrefin (CH)
(74) Representative: P&TS SA (AG, Ltd.)

(56) References cited:
- EP-A1- 2 101 408
- WO-A2-01/54563
- US-B1- 6 366 805

## Description

### Field

The present invention relates to biopotential and/or bioimpedance measurement systems consisting of several units distributed over a human/animal body or body part, and more precisely to the bus connecting these units.

### Description of related art

Biopotential and/or bioimpedance measurements are well known in the art. Biopotentials are for instance ECG or EKG (electrocardiogram), EEG (electroencephalogram), EMG (electromyogram), etc. Bioimpedances are for instance impedance plethysmography (e.g., for respiration), body composition (e.g., hydration, fat, etc.), EIT (electroimpedance tomography) for observations of, e.g., lungs, heart, brain, and muscles.

The classical approach for biopotential and bioimpedance measurements consists of a number of (most often adhesive gel) electrodes placed at specific locations of the body, and shielded electrical wires connecting them to a central unit comprising the electronics. Another approach uses active electrodes, meaning that part of the electronics (usually a pre-amplifier) is placed at the electrodes. All electronics can also be distributed over the electrodes and make units (or electrode-sensors) connected together by electrical connections. When each unit has its own battery, it is possible to reduce the number of electrical connections connecting the units down to one as described in US Patent Application 2011/0001497 A1 by the present applicant. At any given time, one unit plays the role of reference unit while all others are measuring units.

As all measuring units have to be kept isochronously synchronized with each other (so that they can use isochronous demodulation and synchronously acquire all signals), US2011/0001497 A1 proposes a mechanism consisting of periodically reassigning the role of reference unit to each units in turn, because the unit playing the role of reference unit can be synchronized with any selected measuring unit.

The approach proposed in US2011/0001497 A1 for communication is wireless. As a minimum, communication to a centralized receiver (which can possibly be one of the units, or an extra device) is essential to process and exploit the measured signals.

The approach presented in US2011/0001497 A1 allows one measuring unit to inject a current through the body and to ensure its return through the reference unit and shared electrical connection between units. In theory, another measuring unit can also ensure the return of the current by injecting the opposite current, but in practice, as it is impossible to perfectly match two current sources, there will always be a leakage current flowing through the reference unit. Other systems for measuring biopotential and/or bioimpedance of a human or animal body are known from WO01/54563 and US6366805.

### Summary

An object of the invention is to provide, to biopotential and/or bioimpedance measurement systems consisting of a reference unit and one or more measuring units connected together to a bus of one or more electrical conductors,
means for simultaneous and continuous isochronous synchronization of all units, and
means for wired communication between units, and/or
means for adjustment of injected currents of two measuring units so that the leakage current through the reference unit is controlled to zero, and/or
means for simultaneous recharging of all units.

The synchronization mechanism described in US2011/0001497 A1 is complicated and not simultaneous nor continuous. The invention provides a simpler mechanism able to simultaneously and continuously isochronously synchronize all units.

The drawback of wireless communication as proposed in US2011/0001497 A1 is its relatively high power consumption and the high absorption of the body that makes communication difficult when the body is between the emitter and the receiver. The invention proposes a way to communicate between units through the electrical connection(s). Another advantage not to rely on wireless communication is the possibility to operate in some environments that forbid or strongly regulate the use of wireless communications (such as hospitals, airplanes, space vehicles, etc.).

Moreover, the technology presented in US2011/0001497 A1 cannot guarantee that a current can circulate through two measuring units without that a leakage current flows through the reference unit. This may be a problem for, e.g., EIT. The reference unit of the invention can measure the leakage current and control the current sources of the measuring units so that the leakage current is set to zero.

Finally, the system presented in US2011/0001497 A1 assumes that the units are removed from the garment for recharge. When recharging the batteries is needed or when the garment has to be washed, it may not be practical to physically remove a few dozens of units from their garment to connect them to the charger while washing the garment. Another alternative is to make the units watertight and wash the garment with the units together. This has the other advantage to clean the units at the same time, which saves time and guarantees a good hygiene. The invention allows this approach by providing a way to simultaneously recharge all units without removing them from the garment.

Therefore, the invention provides a system for measuring biopotentials and/or bioimpedances of a human or animal body as defined in claim 1.

In another embodiment, at least one measuring unit (2) uses some or all information and/or synchronization signals sent by the reference unit (1) to isochronously operate with the reference unit (1).

In another embodiment, the synchronization signal sent by the reference unit (1) is a periodic signal.

In another embodiment, the synchronization signal sent by the reference unit (1) feeds a phase-lock loop (PLL) in at least one measuring unit (2).

In another embodiment, the information signal sent by the reference unit (1) is transmitted with a self-clocking encoding, such as Manchester code.

In another embodiment, the self-clocking information signal sent by the reference unit (1) is used by at least one measuring unit (2) to regenerate a synchronization signal in phase with the clock of the reference unit (1).

In another embodiment, the information signal sent by the reference unit (1) includes at least one time marker to isochronously synchronize at least one measuring unit (2) with respect to the reference unit (1).

In another embodiment, at least one measuring unit (2) forces the circulation of at least a current i1 from the body surface (4) to at least one bus conductor (3) according to information signals to be transmitted to the reference unit (1) or to at least one other measuring unit (2').

In another embodiment, the reference unit (1) converts to at least one potential u0 to be transmitted back to any other measuring unit (2') the information sent by any measuring unit (2) consisting of at least one sensed current i0 flowing from at least one bus conductor (3) to the body surface (4).

In another embodiment, the conversion is passively performed with one or more impedances inserted in series with at least one bus conductor (3).

In another embodiment, the reference unit (1) senses, in order to acquire the transmitted information sent by any measuring unit (2), at least one sensed current i0 flowing from at least one bus conductor (3) to the body surface (4).

In another embodiment, the reference unit (1) senses, in order to adjust or control the currents forced by the measuring units (2) to measure bio.mpedances, at least one sensed current i0 flowing from at least one bus conductor (3) to the body surface (4).

In another embodiment, any measuring unit (2) forces the circulation of at least a first current i1 from the body surface (4) to at least one bus conductor (3) in order to measure a bioimpedance;
any other measuring unit (2') forces the circulation of at least a second current i2 from the body surface (4) to the same bus conductor (3);
the sensed current i0 flowing from the same bus conductor (3) to the body surface (4) below the reference unit (1) is set to a desired value via a controller controlling the second current i2.

In another embodiment, the desired value is zero.

In another embodiment, each unit has a floating power supply, such as rechargeable battery and/or energy harvesting device.

In another embodiment, at least one bus conductor (3) is further used to power the recharge of the batteries of the units when the system is not operating and removed from the body.

In another embodiment, one of the two conductors required to power the recharge of the batteries is part of the recharging system, the recharge current flowing via one or more bus conductors (3) and the unit electrodes or any other unit non-insulated terminal.

In another embodiment, a conductive liquid, in which the system is immersed, is used as one of the two conductors required to power the recharge of the batteries; and the other conductor is any insulated bus conductor (3)

In another embodiment, the number of bus conductors (3) is one.

In another embodiment, one bus conductor (3) is used for the current i1 forced to circulate for bioimpedance measurement and another bus conductor (3') is used for sensing the corresponding sensed voltage v1.

In another embodiment, one bus conductor (3) is used for the injected current i1 carrying information sent by measuring units (2) and for the set potential u0 carrying information and/or synchronization signals sent by reference unit (1) whereas another bus conductor (3') is used as EMC shield, the bus conductor (3) being between the body surface (4) and the other shielded bus conductor (3') which is preferably shaped as a surface covering the bus conductor (3).

In another embodiment, the information signals sent by the measuring units (2) do not force any current to flow through the body.

In another embodiment, the information signals sent by at least one measuring unit (2) drive a current i1 injected in a bus conductor (3) and the opposite current j1 to another bus conductor (3').

In another embodiment, at least one of the bus conductor (3) is unshielded.

In another embodiment, at least one of the bus conductor (3) is non-insulated.

In another embodiment, at least one of the bus conductor (3) is made of conductive fabrics.

### Brief Description of the Drawings

Figure 1 Generic circuit of the invention
Figure 2 Example of spectrum usage for multiplexing of biopotential, bioimpedance, communication and clock (synchronization) information
Figure 3 Variant that uses the top conductive layer (3') to shield the communication line (3)
Figure 4 Variant that uses two current sources (21) and (21') with j1 = -i1 for communication band (102) and j1 = 0 for the other band, so that no communication current flows through body
Figure 5 Implementation example of simultaneous recharge of all units

### Detailed Description of possible embodiments

The present invention will be described in more detail below. It is understood that the various embodiments, preferences and ranges as provided/disclosed in this specification may be combined at will. Further, depending of the specific embodiment, selected definitions, embodiments or ranges may not apply.

As used in this specification and the appended claims, the singular forms "a", "an", and "the" encompass embodiments having plural referents, unless the content clearly dictates otherwise.

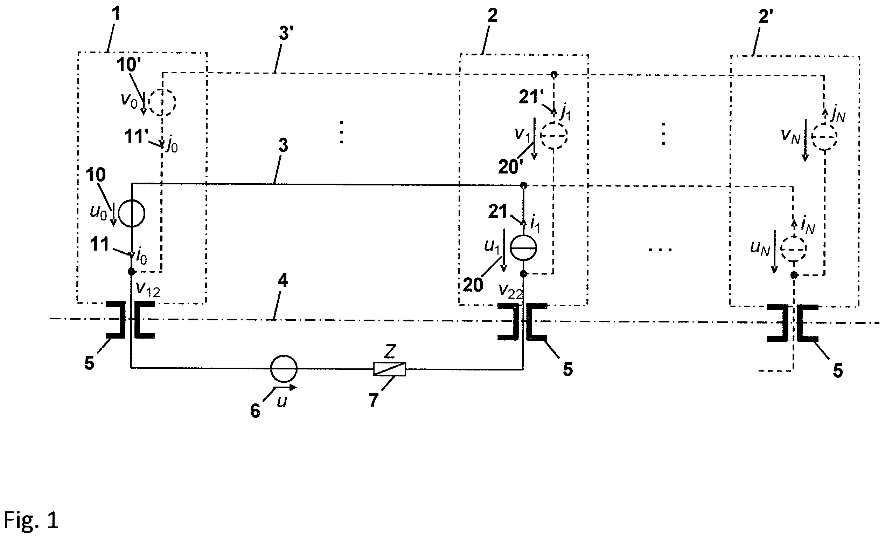

Figure 1 shows the generic circuit of the invention. The reference unit (1) and one or more measuring unit (2, 2', ...) are connected together with one or more electrical connection (3, 3', ...) and placed on the skin (4). The frontend circuit is symbolized by the pass-through (5), since its purpose is precisely to virtually bring a direct connection through the high impedance of the skin stratum corneum.

The biopotential u (6) is measured by acquiring the voltage u1 (20) or v1 (20'). For example, each lead ECG is measured between the reference unit (1) and a given measuring unit (2). If a lead between two measuring units is desired, it can simply be obtained by making the arithmetic difference between the two measured leads.

The bioimpedance Z (7) is measured by injecting the current i1 (21) or j1 (21') and by acquiring the voltage u1 (20) or v1 (20'). Notice that the impedance measurement is generally measured at high frequency (e.g., 50 kHz) to avoid any interference with the biopotentials and because at high frequency, the skin impedance is lower and more stable.

In its simpler representation, the invention consists of
voltage sources u0 (10) or v0 (10') of the reference unit (1) broadcasting to all measuring units (2, 2', ...) clock signals or equivalent so that the measuring units can acquire them through the measurement of the voltages u1 (20) or v1 (20');
current sources i1 (21) or j1 (21') of the measuring units (2, 2', ...) driven to send data to the reference unit (1) which receives them by acquiring the current i0 (11) or j0 (11');
voltage sources u0 (10) or v0 (10') of the reference unit (1) broadcasting to all measuring units (2, 2', ...) data received by the measuring units acquiring them through the measurement of the voltages u1 (20) or v1 (20');
controllers (not represented in Figure 1) that control the current sources i1 (21) or j1 (21') so that the leakage currents i0 (11) or j0 (11') are null.

These controllers can be in the reference unit, in the measuring units, or somewhere else. The input and output signals of the controllers must be transmitted for example by the communication means described above or wirelessly.

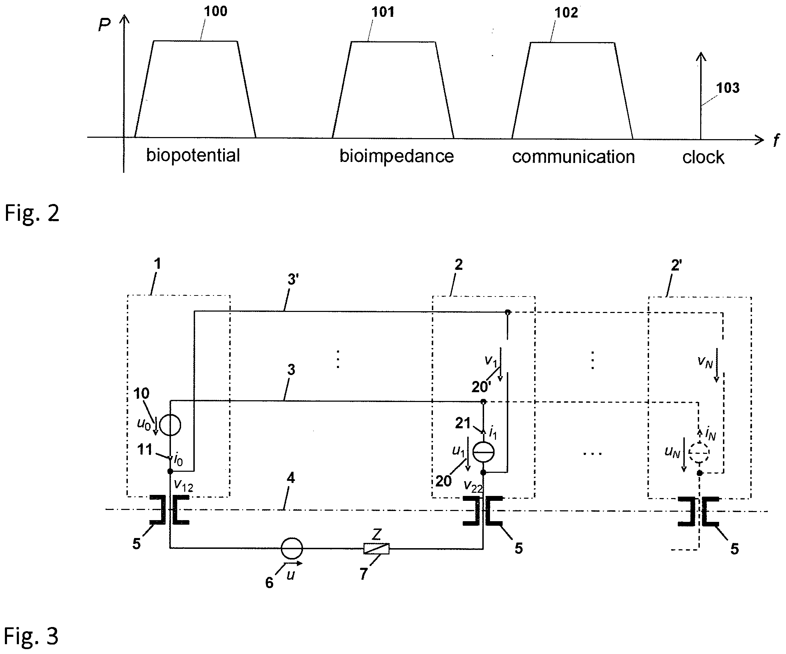

As the voltage and current sources as well as the voltage and current measurements described above can play several roles, a given frequency band is assigned for each role as shown in Figure 2. The low-frequency band (100), e.g., from 0.05 to 40 Hz for ECG, is reserved for biopotential measurements. The medium band (101), e.g., around 50 kHz, is assigned to bioimpedance measurements (with several subcarriers for each current injection configuration). The full-duplex communication between the reference unit (1) and the measuring units (2, 2', ...) can use the highfrequency band (102), e.g., around 1 MHz.

The master clock (103) necessary for synchronous communication is generated in the reference unit (1). An explicit clock (103) is however not necessary to be transmitted if self-clocking encoding is used for the communication information (102) emitted by the reference unit (1). For example, for a Manchester keying, enough clock information is contained in the signal. In practice, it may be therefore more advantageous not to explicitly transmit the clock (103), even though the clock recovery from a Manchester keying is more difficult than a simple PLL.

Note that except for the biopotential band (100), other type of multiplexing than frequency multiplexing (e.g., time multiplexing) can be used in the remaining frequency band for all the other signals.

Figure 3 shows another embodiment of the invention that uses two electrical connections. The advantage of this embodiment is to separate the current path (3) from the voltage path (3'). This may be important when measuring the impedance (7), in particular when the impedance of the electrical connections (3) and (3') varies, for instance if these connections are made of conductive textile.

For EMC reasons, it may be preferable to shield the communication line. One way to achieve this is also shown in the embodiment of Figure 3 where the top conductive layer (3') is used to shield the communication line (3) at the band (102, 103). Line (3') is still used for the biopotential band (100) and bioimpedance band (101) as voltage path.

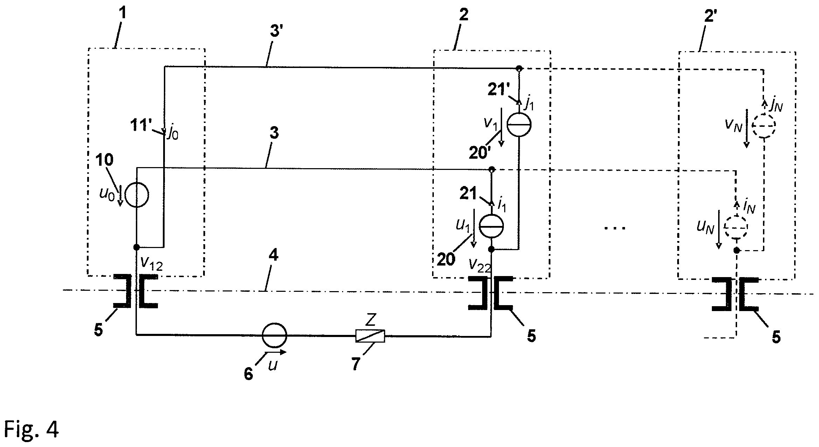

Figure 4 shows another embodiment characterized in that the communication current is injected by the current source (21) to the line (3), and return by the line (3') before being neutralized by the current source (21') which inject an opposite current. This way, no current (for communication) flows through the skin (4). Note that for the other bands (100, 101), the current source (21) is the only one that injects the current (for these bands, the current source 21' is off).

In a preferred embodiment, each unit shall also comprise signal acquisition means in order to sense voltages (21, 21') and currents (11, 11'). This typically implies amplification and transimpedance electronics, respectively, possibly band filtering and demodulation electronics, anti-aliasing filtering, and a microcontroller with ADCs (analogue to digital converters). The (signal) data acquired by the microcontroller can be further processed (e.g., separation/demodulation, digital filtering, signal exploitation, etc.) and some data can be transmitted to other units or preferably to the reference unit (1) for global processing.

Note that in some situations, it may be advantageous not to pass by a microcontroller. For example, if a measuring unit would like to transmit information to another measuring unit, the data have to be sent to the reference unit (1) which will broadcast them to all measuring unit (2). To do this, a simple impedance (typically an inductance in parallel with a resistance) at the reference unit will directly translate the arriving data encoded in the modulated current i0 (11) or j0 (11') into the broadcast data encoded in the modulated voltage u0 (10) or v0 (10'). Therefore, no microcontroller is needed for this relay operation.

Other physiological or environmental signals than biopotential and bioimpedance are of interest and can easily be added to the units, for example
accelerometer, gyroscope, magnetometer, etc. (e.g., to measure activity and posture),
single- or multi-frequency LED/PD photo-plethysmography (e.g., to measure SpO2, SpCO, perfusion, etc.),
ultra-, infra-, or sound transducer (e.g., to measure heart sounds or to perform ultrasound tomography),
temperature sensor (e.g., to directly measure skin temperature and indirectly measure core body temperature),
sweat sensor, CO2/O2 sensor,
environment sensors (ambient air temperature, humidity, and pressure; gas composition, etc.),
ingested smart pill and medication-compliance sensor

The units may as well:
apply voltage and inject current for defibrillation
apply voltage and inject current for skeletal-muscle stimulation

In the preferred embodiment, the reference unit (1) and every measuring unit (2) have their own power supply, which can be a primary or rechargeable battery, or some energy harvesting system, such as solar cell, electro-thermal transducer (such as thermopile), etc. Each unit has therefore its own ground (i.e., independent floating power supplies).

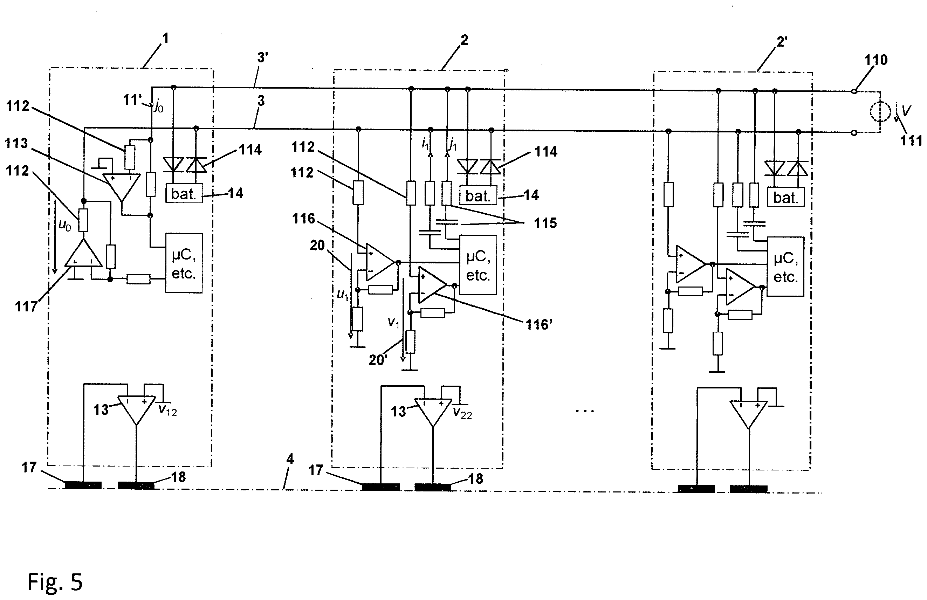

Figure 5 shows an implementation example of the pass-through circuit (5) which is possible thanks to floating power supplies of each unit. The pass-through circuit is realized with an operational amplifier (13), powered by a voltage source (14) and driving the electrode (18) according to the voltage feedback measured at electrode (17). Therefore, the unit ground is equal to the potential measured at electrode (17), which is itself equal to the potential beyond the high impedance of the skin, because no current flows via the electrode (17). The pass-through circuit separates the voltage and current paths and uses two electrodes for this purpose. Therefore, the current i0 (11) or i1 (21) flows via the electrode (18), and the voltage u1 (20) is equal to the voltage between the electrodes (17) of the reference and measuring units plus the voltage u0 (10).

Figure 5 also shows an implementation example that allows sharing the two electrical connections during measurement (mode 1) and during recharge (mode 2). During measurement, the power supply (111) is disconnected from the terminal (110). Therefore, there is no safety issue even if the electrical connections (3, 3') are poorly insulated. Moreover, the electrical connections (3, 3') are entirely for the measurement function.

The recharge is performed during maintenance, when the system is not on the body. In this mode, the power supply (voltage source 111) is connected to the terminal (110) and distributes the power to all units simultaneously. As the voltage V (111) is higher than the unit battery voltage, the unit battery (14) can be recharged through the diodes (114). Note that these diodes (114) prevent any power to come out the battery (14). Therefore, during measurement, the diodes (114) isolate the battery (14) from the bus (3, 3').

Because during recharge, the voltage on the bus (3, 3') is higher than the voltage of the battery (14), resistances (112) are added to prevent a too high leakage current to flow through the protection diodes of the ICs.

In this example, the voltage source u0 (10) is realized by the operational amplifier (117) mounted in a voltage amplifier (with a negative gain that can as well be smaller than 1, depending on the choice of the two resistances connected to its negative input).

The current sensing j0 (11') is performed thanks to the operational amplifier (113) and its negative feedback resistance. This circuit actually realizes a negative transimpedance: the current flows towards a virtual ground and is transformed into voltage by the resistance.

The operational amplifiers (116) and (118) are, with their resistances, voltage amplifiers to sense the voltages u1 (20) and v1 (20').

Finally, the current sources i1 (21) and j1 (21') are simply realized by a relatively high impedance (115) in series with the voltage source of the microcontroller (µC).

In another embodiment, at least one of the electrical connection between the units is insulated and watertight altogether with the units. A voltage applied between this insulated connection and the washing water (or any other conductive liquid) will allow recharging all units via one of their electrodes or any non-insulated contact.

### Reference numbers

- 1: reference unit
- 2: measuring unit
- 2': other measuring unit
- 3: bus conductor
- 3': other bus conductor
- 4: body surface (e.g., skin)
- 5: pass-through circuit
- 6: biovoltage source
- 7: bioimpedance
- 10: voltage source
- 10': other voltage source
- 11: sensed current i0
- 11': other sensed current j0
- 13: pass-through operational amplifier
- 14: rechargeable battery
- 17: voltage-sensing electrode
- 18: current-injecting electrode
- 20: sensed voltage u1
- 20': other sensed voltage v1
- 21: current source
- 21': other current source
- 100: biopotential band
- 101: bioimpedance band
- 102: communication band
- 103: clock spectral line
- 110: bus terminals
- 111: recharging voltage source
- 112: protective resistance
- 113: current-to-voltage converter
- 114: recharging diodes
- 117: voltage amplifier as voltage source (10)
- 115: high impedance converting voltage to current source
- 116: voltage amplifier of sensed voltage (20)
- 116': voltage amplifier of other sensed voltage (20')
- f: frequency
- u: biovoltage of (6)
- u0: voltage of (10)
- v0: voltage of (10')
- v12: reference potential of reference unit (1)
- v22: reference potential of measuring unit (2)
- i1: current of (21)
- j1: current of (21')
- P: power
- V: voltage of recharging power source (111)
- Z: bioimpedance

## Claims

1. A system for measuring biopotential and/or bioimpedance of a human or animal body comprising:
a reference unit (1) adapted to be placed on the body surface (4);
a measuring unit (2) also adapted to be placed on the body surface (4); and
a bus conductor (3) connecting the units (1, 2);
the units (1, 2) being connected in parallel by the bus conductor (3);
**characterized in that**
the reference unit (1) comprises a voltage source (10) for setting a potential (u0) of said bus conductor (3) with respect to a reference potential (v12) of the reference unit (1) according to information and/or synchronization signals to be transmitted to the measuring units (2), the reference potential (v12) of the reference unit (1) being substantially equal to the potential of the body surface (4) when the reference unit (1) is placed on the body surface (4) so that the information and/or synchronization signals are carried by the potential (u0) and transmitted from the reference unit (1) to the measuring units (2) via the bus conductor (3); and **in that**
said measuring unit (2) is further electrically connected to the bus conductor (3) so that it can sense the potential (u1) of said bus conductor with respect to the reference potential (v22) of the measuring unit (2) in order to acquire the transmitted information and/or synchronization signals sent by the reference unit (1).

2. A system according to claim 1, wherein
said measuring unit (2) is configured to receive
said information and/or synchronization signals sent by the reference unit (1) and is configured to isochronously operate with the reference unit (1).

3. A system according to claim 2, wherein
the synchronization signal sent by the reference unit (1) is a periodic signal feeding a phase-lock loop (PLL) in said measuring unit (2).

4. A system according to claim 2, wherein
the information signal sent by the reference unit (1) can be transmitted with a self-clocking encoding and used by said measuring unit (2) to regenerate a synchronization signal in phase with the clock of the reference unit (1).

5. A system according to any one of claim 2 to 4, wherein
the information signal sent by the reference unit (1) includes a time marker to isochronously synchronize said measuring unit (2) with respect to the reference unit (1).

6. A system according to claim 2 or 5, wherein
said measuring unit (2) is further arranged to force the circulation of at least a current (i1) from the body surface (4) to said bus conductor (3) according to information signals to be transmitted to the reference unit (1) or to said other measuring unit (2').

7. A system according to claim 6, wherein
the reference unit (1) is further arranged for converting the information sent by any measuring unit (2) and received by the reference unit (1) as a sensed current (i0) flowing from said bus conductor (3) to the body surface (4) when the measuring unit (2) is placed on the body surface (4), to said potential (u0) to be transmitted back to any other measuring unit (2').

8. A system according to claim 6 or 7, wherein
the reference unit (1) is further arranged for sensing, a sensed current (i0) flowing from said bus conductor (3) to the body surface (4), when the measuring unit (2) is placed on the body surface (4), in order to acquire the transmitted information sent by any measuring unit (2).

9. A system according to claim 2, 5, or 8, wherein
the reference unit (1) is further arranged for sensing, said sensed current (i0) flowing from a bus conductor (3) to the body surface (4), when the reference unit (1) is placed on the body surface (4) in order to adjust or control the currents forced by the measuring units (2) to measure bioimpedances.

10. A system according to claim 9, wherein
any one of said measuring unit (2) is adapted to force the circulation of at least a first current (i1) from the body surface (4) to said bus conductor (3) to measure a bioimpedance; and
any other of said measuring unit (2') is adapted to force the circulation of at least a second current (i2) from the body surface (4) to the same bus conductor (3); the sensed current (i0) flowing from the same bus conductor (3) to the body surface (4) below the reference unit (1) is set to a desired value, via a controller controlling the second current (i2).

11. A system according to claim 2, 5, 8, or 10, wherein
said reference unit (1) and said measuring unit (2) comprise a battery, and wherein
said bus conductor (3) being further arranged for powering the recharge of the batteries of the units(1, 2).

12. A system according to claim 1, wherein
said bus conductor (3) is further arranged to be used for forcing said current (i1) to circulate for bioimpedance measurement and another of said bus conductor (3') is further arranged to be used for sensing the corresponding sensed voltage (v1).

13. A system according to claim 1, wherein
said bus conductor (3) is further arranged to be used for the injected current (i1) carrying information sent by said measuring unit (2) and for the set potential (u0) carrying information and/or synchronization signals sent by reference unit (1), whereas another of said bus conductor (3') is further arranged to be used as electromagnetic shield, said bus conductor (3) being between the body surface (4), when said reference unit (1) and said measuring units (2) are placed on the body (4), and the other of said shielded bus conductor (3').

14. A system according to claim 8, wherein
the information signals sent by said measuring units (2) is further arranged such as to not force any current to flow through the body (4), when reference unit (1) and said measuring units (2) are placed on the body (4).

## Patentansprüche

1. System zur Messung von Biopotenzial und/oder Bioimpedanz eines menschlichen oder tierischen Körpers, umfassend:
eine Referenzeinheit (1), die dazu angepasst ist, auf die Körperoberfläche (4) platziert zu werden;
eine Messeinheit (2), die ebenfalls dazu angepasst ist, auf die Körperoberfläche (4) platziert zu werden;
einen Busleiter (3), der die Einheiten (1, 2) verbindet;
wobei die Einheiten (1, 2) durch den Busleiter (3) parallel verbunden sind;
**dadurch gekennzeichnet, dass**
die Referenzeinheit (1) eine Spannungsquelle (10) zum Einstellen eines Potenzials (u0) des Busleiters (3) mit Bezug auf ein Referenzpotenzial (v12) der Referenzeinheit (1) gemäß an die Messeinheiten (2) zu sendenden Informations- und/oder Synchronisationssignalen umfasst, wobei das Referenzpotenzial (v12) der Referenzeinheit (1) im Wesentlichen gleich dem Potenzial der Körperoberfläche (4) ist, wenn die Referenzeinheit (1) auf die Körperoberfläche (4) platziert wird, sodass die Informations- und/oder Synchronisationssignale von dem Potenzial (u0) getragen und von der Referenzeinheit (1) über den Busleiter (3) an die Messeinheiten (2) gesendet werden; und dadurch, dass
die Messeinheit (2) ferner elektrisch mit dem Busleiter (3) verbunden ist, sodass sie das Potenzial (u1) des Busleiters mit Bezug auf das Referenzpotenzial (v22) der Messeinheit (2) erfassen kann, um die Informations- und/oder die Synchronisationssignale zu erhalten, die von der Referenzeinheit (1) gesendet wurden.

2. System nach Anspruch 1, wobei
die Messeinheit (2) dazu ausgelegt ist, die Informations- und/oder Synchronisationssignale zu empfangen, die von der Referenzeinheit (1) gesendet wurden, und dazu ausgelegt ist, isochron mit der Referenzeinheit (1) zu arbeiten.

3. System nach Anspruch 2, wobei
das Synchronisationssignal, das von der Referenzeinheit (1) gesendet wurde, ein periodisches Signal ist, das eine Phasenregelschleife (PLL) in der Messeinheit (2) speist.

4. System nach Anspruch 2, wobei
das Informationssignal, das von der Referenzeinheit (1) gesendet wurde, mit einer selbsttaktierenden Kodierung gesendet und von der Messeinheit (2) genutzt werden kann, um ein Synchronisationssignal phasengleich mit der Referenzeinheit (1) erneut zu erzeugen.

5. System nach einem der Ansprüche 2 bis 4, wobei
das Informationssignal, das von der Referenzeinheit (1) gesendet wurde, eine Zeitmarke umfasst, um die Messeinheit (2) mit Bezug auf die Referenzeinheit (1) isochron zu synchronisieren.

6. System nach Anspruch 2 oder 5, wobei
die Messeinheit (2) ferner dazu angeordnet ist, die Zirkulation von mindestens einem Strom (i1) von der Körperoberfläche (4) an den Busleiter (3) entsprechend der an die Referenzeinheit (1) oder die andere Messeinheit (2') zu sendenden Informationssignale zu forcieren.

7. System nach Anspruch 6, wobei
die Referenzeinheit (1) ferner zur Umwandlung der Informationen angeordnet ist, die von einer Messeinheit (2) gesendet und von der Referenzeinheit (1) empfangen wurden als ein erfasster Strom (i0), der von dem Busleiter (3) an die Körperoberfläche (4) fließt, wenn die Messeinheit (2) auf der Körperoberfläche (4) platziert wird, in das Potenzial (u0), das an eine andere Messeinheit (2') zurückzusenden ist.

8. System nach Anspruch 6 oder 7, wobei
die Referenzeinheit (1) ferner zum Erfassen eines erfassten Stroms (i0) angeordnet ist, der von dem Busleiter (3) an die Körperoberfläche (4) fließt, wenn die Messeinheit (2) auf der Körperoberfläche (4) platziert wird, um die gesendeten Informationen zu erhalten, die von einer Messeinheit (2) gesendet wurden.

9. System nach Anspruch 2, 5 oder 8, wobei
die Referenzeinheit (1) ferner zum Erfassen des erfassten Stroms (i0) angeordnet ist, der von dem Busleiter (3) an die Körperoberfläche (4) fließt, wenn die Referenzeinheit (1) auf der Körperoberfläche (4) platziert wird, um die Ströme einzustellen oder zu steuern, die von den Messeinheiten (2) forciert wurden, um Bioimpedanzen zu messen.

10. System nach Anspruch 9, wobei
eine der Messeinheiten (2) dazu angepasst ist, die Zirkulation von mindestens einem ersten Strom (i1) von der Körperoberfläche (4) an den Busleiter (3) zu forcieren, um eine Bioimpedanz zu messen; und
eine andere der Messeinheiten (2') dazu angepasst ist, die Zirkulation von mindestens einem zweiten Strom (i2) von der Körperoberfläche (4) an denselben Busleiter (3) zu forcieren; wobei der erfasste Strom (i0), der von demselben Busleiter (3) an die Körperoberfläche (4) unter der Referenzeinheit (1) fließt, auf einen gewünschten Wert eingestellt wird, über eine Steuerung, die den zweiten Strom (i2) steuert.

11. System nach Anspruch 2, 5, 8 oder 10, wobei
die Referenzeinheit (1) und die Messeinheit (2) eine Batterie umfassen, und wobei
der Busleiter (3) ferner zur Energieversorgung des Aufladens der Batterien der Einheiten (1, 2) angeordnet ist.

12. System nach Anspruch 1, wobei
der Busleiter (3) ferner dazu angeordnet ist, dazu genutzt zu werden, zu forcieren, dass der Strom (i1) für die Bioimpedanzmessung zirkuliert, und ein anderer Busleiter (3') ferner dazu angeordnet ist, zum Erfassen der entsprechenden erfassten Spannung (v1) genutzt zu werden.

13. System nach Anspruch 1, wobei
der Busleiter (3) ferner dazu angeordnet ist, für den injizierten Strom (i1) genutzt zu werden, der Informationen trägt, die von der Messeinheit (2) gesendet wurden, und für das eingestellte Potenzial (u0), Informations- und/oder Synchronisationssignale, die von der Referenzeinheit (1) gesendet wurden, trägt, wohingegen ein anderer Busleiter (3') ferner dazu angeordnet ist, als elektromagnetische Abschirmung genutzt zu werden, wobei der Busleiter (3), der sich zwischen der Körperoberfläche (4), wenn die Referenzeinheit (1) und die Messeinheiten (2) auf dem Körper (4) platziert sind, und dem anderen abgeschirmten Busleiter (3') befindet.

14. System nach Anspruch 8, wobei
die Informationssignale, die von den Messeinheiten (2) gesendet wurden, ferner dazu angeordnet sind, nicht zu forcieren, dass ein Strom durch den Körper (4) fließt, wenn die Referenzeinheit (1) und die Messeinheiten (2) auf dem Körper (4) platziert werden.

## Revendications

1. Système de mesure du biopotentiel et/ou de la bioimpédance d'un corps humain ou animal comprenant :
une unité de référence (1) adaptée pour être placée sur la surface du corps (4) ;
une unité de mesure (2) également adaptée pour être placée sur la surface du corps (4) ; et
un conducteur de bus (3) connectant les unités (1, 2) ;
les unités (1, 2) étant connectées en parallèle par le conducteur de bus (3) ;
**caractérisé en ce que**
l'unité de référence (1) comprend une source de tension (10) pour régler un potentiel (u0) dudit conducteur de bus (3) par rapport à un potentiel de référence (v12) de l'unité de référence (1) en fonction des signaux d'information et/ou de synchronisation devant être transmis aux unités de mesure (2), le potentiel de référence (v12) de l'unité de référence (1) étant sensiblement égal au potentiel de la surface du corps (4) lorsque l'unité de référence (1) est placée sur la surface du corps (4) de sorte que les signaux d'information et/ou de synchronisation sont transportés par le potentiel (u0) et transmis de l'unité de référence (1) aux unités de mesure (2) via le conducteur de bus (3) ; et **en ce que**
ladite unité de mesure (2) est en outre connectée électriquement au conducteur de bus (3) de sorte qu'elle peut détecter le potentiel (u1) dudit conducteur de bus par rapport au potentiel de référence (v22) de l'unité de mesure (2) afin d'acquérir les informations transmises et/ou les signaux de synchronisation envoyés par l'unité de référence (1).

2. Système selon la revendication 1,
ladite unité de mesure (2) étant configurée pour recevoir lesdites informations et/ou signaux de synchronisation envoyés par l'unité de référence (1) et étant configurée pour fonctionner de manière isochrone avec l'unité de référence (1).

3. Système selon la revendication 2,
le signal de synchronisation envoyé par l'unité de référence (1) étant un signal périodique alimentant une boucle à verrouillage de phase (PLL) dans ladite unité de mesure (2).

4. Système selon la revendication 2,
le signal d'information envoyé par l'unité de référence (1) pouvant être transmis avec un codage à auto-synchronisation et utilisé par ladite unité de mesure (2) pour régénérer un signal de synchronisation en phase avec l'horloge de l'unité de référence (1).

5. Système selon l'une quelconque des revendications 2 à 4,
le signal d'information envoyé par l'unité de référence (1) comprenant un marqueur temporel pour synchroniser de manière isochrone ladite unité de mesure (2) par rapport à l'unité de référence (1).

6. Système selon la revendication 2 ou 5,
ladite unité de mesure (2) étant en outre agencée pour forcer la circulation d'au moins un courant (i1) de la surface du corps (4) vers ledit conducteur de bus (3) en fonction de signaux d'information devant être transmis à l'unité de référence (1) ou à ladite autre unité de mesure (2').

7. Système selon la revendication 6,
l'unité de référence (1) étant en outre agencée pour convertir les informations envoyées par n'importe quelle unité de mesure (2) et reçues par l'unité de référence (1) comme un courant détecté (i0) s'écoulant dudit conducteur de bus (3) vers la surface du corps (4) lorsque l'unité de mesure (2) est placée sur la surface du corps (4), en ledit potentiel (u0) devant être transmis à n'importe quelle autre unité de mesure (2').

8. Système selon la revendication 6 ou 7,
l'unité de référence (1) étant en outre agencée pour détecter un courant détecté (i0) s'écoulant dudit conducteur de bus (3) vers la surface du corps (4), lorsque l'unité de mesure (2) est placée sur la surface du corps (4), afin d'acquérir les informations envoyées par n'importe quelle unité de mesure (2).

9. Système selon la revendication 2, 5 ou 8,
l'unité de référence (1) étant en outre agencée pour détecter ledit courant détecté (i0) s'écoulant d'un conducteur de bus (3) vers la surface du corps (4), lorsque l'unité de référence (1) est placée sur la surface du corps (4) afin d'ajuster ou commander les courants forcés par les unités de mesure (2) pour mesurer les bioimpédances.

10. Système selon la revendication 9,
l'une quelconque de ladite unité de mesure (2) étant adaptée pour forcer la circulation d'au moins un premier courant (i1) de la surface du corps (4) vers ledit conducteur de bus (3) pour mesurer une bioimpédance ; et
n'importe quelle autre desdites unités de mesure (2') étant adaptée pour forcer la circulation d'au moins un second courant (i2) de la surface du corps (4) vers le même conducteur de bus (3) ; le courant détecté (i0) s'écoulant du même conducteur de bus (3) vers la surface du corps (4) sous l'unité de référence (1) étant réglé à une valeur souhaitée, via un dispositif de commande commandant le second courant (i2).

11. Système selon la revendication 2, 5, 8 ou 10,
ladite unité de référence (1) et ladite unité de mesure (2) comprenant une batterie, et
ledit conducteur de bus (3) étant en outre agencé pour alimenter la recharge des batteries des unités (1, 2) .

12. Système selon la revendication 1,
ledit conducteur de bus (3) étant en outre agencé pour être utilisé pour forcer ledit courant (i1) à circuler pour une mesure de bioimpédance et un autre dudit conducteur de bus (3') étant en outre agencé pour être utilisé pour détecter la tension mesurée correspondante (v1).

13. Système selon la revendication 1,
ledit conducteur de bus (3) étant en outre agencé pour être utilisé pour le courant injecté (i1) transportant des informations envoyées par ladite unité de mesure (2) et pour le potentiel réglé (u0) transportant des informations et/ou des signaux de synchronisation envoyés par l'unité de référence (1), tandis qu'un autre dudit conducteur de bus (3') est en outre agencé pour être utilisé comme protection électromagnétique, ledit conducteur de bus (3) se trouvant entre la surface du corps (4), lorsque ladite unité de référence (1) et lesdites unités de mesure (2) sont placées sur le corps (4), et l'autre dudit conducteur de bus protégé (3').

14. Système selon la revendication 8,
les signaux d'information envoyés par lesdites unités de mesure (2) étant en outre agencés de manière à ne pas forcer un courant à s'écouler à travers le corps (4), lorsque l'unité de référence (1) et lesdites unités de mesure (2) sont placées sur le corps (4).
